# EUROPÄISCHE PATENTSCHRIFT

(11) **EP 1 542 644 B1**
(45) Veröffentlichungstag und Bekanntmachung des Hinweises auf die Patenterteilung: **05.12.2007**
(21) Anmeldenummer: 03773458.9
(22) Anmeldetag: 24.09.2003
(51) Int. Cl.: A61J 7/00, A61M 15/00, A61M 5/172

(54) **MEDIKAMENT-DOSIMETER-KOMBIPACKUNG**
MEDICAMENT/DOSIMETER COMBINATION PACKAGING
ENSEMBLE COMBINE MEDICAMENT-DOSIMETRE

(30) Priorität: 27.09.2002 DE 10245508
(43) Veröffentlichungstag der Anmeldung: 22.06.2005
(73) Patentinhaber: MCS Micro Carrier Systems GmbH, 41460 Neuss (DE)
(72) Erfinder: SCHREIER, Hans, 40545 Düsseldorf (DE); GREB, Wolfgang, 40489 Düsseldorf (DE)
(74) Vertreter: Christophersen, Ruth
(86) Internationale Anmeldenummer: PCT/DE2003/003186
(87) Internationale Veröffentlichungsnummer: WO 2004/030604

(56) Entgegenhaltungen:
- EP-A- 0 689 848
- EP-A- 0 777 123
- WO-A-03/043684

## Beschreibung

Die vorliegende Erfindung betrifft ein System für die individuelle Dosierung eines Arzneistoffs, entsprechend den individuellen phathologischen, dem Krankheitszustand entsprechenden physiologischen, bzw. genetischen Eigenschaften ('fingerprint') eines Patienten. Das System besteht aus zwei Komponenten, dem Arzneistoff, der in variabler individueller Dosierung eingenommen, bzw. verabreicht wird, und einem miniaturisierten Anzeigesystem, das Informationen aus Blut-, Speichel-, oder anderen Körperflüssigkeiten und Geweben des Patienten gewinnt und lesbar darstellt, so dass der Patient oder der behandelnde Arzt daraus sofort die optimal einzunehmende, bzw. zu verabreichende Dosierung ersehen kann.

Die Erforschung des menschlichen Genoms und die damit einhergehende Identifizierung einer grossen Anzahl von Genen (Wirkorte) hat auch die diagnostische Medizin revolutioniert. Die genetischen Grundlagen vieler im Körper ablaufenden Prozesse, z.B. von Enzymen gesteuerte metabolische Prozesse, sind bereits bekannt. Gene regulieren auch die Aktivität von zellulären Enzymen, die u.a. die Verstoffwechselung, Resorption und Wirkung bzw. Nebenwirkung von Medikamenten individuell bestimmen. Darüberhinaus werden immer mehr genetische Mutationen und die daraus resultierenden Defekte, bzw. Erkrankungen, mit relativ schnellen und genauen Methoden erkannt. Diese Information über die individuelle Charakteristik der Zellaktivität eines Patienten nutzt die behandelnde Medizin für die optimale Anwendung eines entsprechenden Arzneimittels bezüglich, Typ, Dosierung und Dosierungsinterval eines entsprechenden Arzneimittels.

Gegenstand der vorliegenden Erfindung ist eine Medikament-Dosimeter-Kombipackung, die in einer Verpackung
a. ein individuell dosierbares Arzneimittel, und
b. ein diagnostisches Anzeigesystem für einen körpereigenen Stoff, Regulationsmechanismus oder Gen oder Indikationssystem, relevant für die Wirkung, Nebenwirkung, Interaktion, Stoffwechsel, Absorption, Verteilung, Metabolismus und Elimination des zu verabreichenden Arzneistoffes
enthält.

Das erfindungsgemäße System eignet sich insbesondere für die individuelle Dosierung eines Arzneistoffs, entsprechend den individuellen pathologischen und physiologischen, bzw. genetischen Eigenschaften ('fingerprint') eines Patienten. Das System besteht aus zwei Komponenten, dem Arzneistoff, der in variabler individueller Dosierung eingenommen, bzw. verabreicht wird, und einem miniaturisierten Anzeigesystem, das Informationen aus Blut-, Speichel-, oder anderen Körperflüssigkeiten und Geweben des Patienten gewinnt und lesbar darstellt, so dass der Patient oder der behandelnde Arzt daraus sofort die optimal einzunehmende, bzw. zu verabreichende Dosierung ersehen kann. In speziellen Fällen ist daraus auch zu sehen, ob mit der Einnahme eines bestimmten Medikaments überhaupt ein therapeutischer Effekt erzielt werden kann (Responder / Non-Responder Definition).

Der Einsatz der vorliegenden Erfindung findet auf drei Ebenen statt:
1. als analytische Messeinheit vor Einnahme, bzw. Dosierung eines Medikaments, um den (genetischen) Typ des Patienten zu definieren und daraus den Schluss zu ziehen, ob der Patient mit einem spezifischen Medikament oder mit einer speziellen Menge eines Medikaments behandelt werden soll oder nicht;
2. als Dosierungsmesseinheit während der Medikation, um dem Patienten kontinuierlich die optimale Dosis Arzneistoff zur Verfügung zu stellen;
3. als Monitoringmesseinheit, die den Effekt einer Medikation kontinuierlich misst und dokumentiert und damit dem Patienten und/oder behandelnden Arzt erlaubt, Erfolg oder Misserfolg einer Medikation kontinuierlich zu verfolgen.

Während der Arzneistoff selbst in jeder beliebigen pharmazeutischen Form, als Lösung, Tropfen, Tablette, Micropellets, Creme, Inhalat, etc. vorliegen kann, wird das Anzeigesystem im einfachsten Fall aus einem Papier- oder Plastikstab bestehen, an dessen Ende sich eine reaktive Zone befindet, die mit der Körperflüssigkeit in Kontakt gebracht wird. Dies kann entweder ein mit einem Reaktionsgemisch getränkter Papierstreifen, oder eine Vertiefung (Welle), oder ein Gefäss mit Indikatorlösung oder Reaktionslösung sein, in der die Körperflüssigkeit und eine chemische Flüssigkeit vermischt werden. Das Ergebnis der Reaktion (Signal) wird im einfachsten Fall als Farbumschlag angezeigt und kann als solches dem Patienten, bzw. Arzt einen Bereich, bzw. eine Ausschlussgrenze signalisieren, die dieser dann zur Entscheidung über die einzunehmende Menge Arzneistoff benutzen kann.

Dem Testsystem kann aber auch eine quantitative chemische Reaktion zugrundeliegen, deren Ergebnis nicht nur mit dem blossen Auge abgeschätzt werden kann, sondern nach Einführen des Teststäbchens oder der Testwelle in ein entsprechendes analytisches Gerät ('Anzeiger') quantitativ als definierter Wert erfasst und gelesen werden kann. Das analytische Gerät seinerseits kann wiederum an eine Datenbank angeschlossen sein, worin historische Daten des Patienten bereits gespeichert sind und somit weiter zur Entscheidungsfindung herangezogen werden können.

Ein Testsystem kann aber auch aus einem Chip bestehen, der mit einer oder mehreren reaktiven Substanzen beschichtet ist und nach Reaktion mit der aufgebrachten Körperflüssigkeit eine oder mehrere messbare Resultate liefert und Dosierungen vorschlägt, oder erlaubt, bzw. ausschliesst.

Im Idealfall lassen sich Testsystem und Arzneiform so miteinander vernetzen, dass durch Zusammenbringen des Chip mit einem entsprechenden Chip auf oder in der Arzneiform letztere ohne Zutun des Patienten oder Arztes auf Grund der ihr zur Verfügung gestellten Information die für jeden gegebenen Zustand optimale Dosis des Arzneistoffs benennt oder freigibt. Beispiele dafür sind eine Kasette, die entsprechend der abgelesenen Information eine bestimmte Menge Kapseln oder Tabletten freigibt, ein programmierter Tropfenspender oder Cremespender, eine subkutane Injektion z.B. mit einem 'Pen-Injector', der eine genau definierte, aber jedesmal je nach individueller Information variable Menge Arzneistoff unter die Haut injiziert, oder ein variabler Vernebler, der die der Information entsprechende Menge Substanz vernebelt, die der Patient dann inhaliert.

Mögliche Ausführungsformen der Erfindung schliessen folgende Kombinationen ein.

Die Medikament-Dosimeter-Kombipackung hat im einfachsten Fall zwei separate Fächer in denen im einen der Arzneistoff in einer bestimmten Darreichungsform, und im anderen eine entsprechende Anzahl Teststreifen untergebracht sind. In diesem Fall ist es in der Hand des Patienten, bzw. Arztes, das Anzeigesystem vor Gabe einer entsprechenden Dosis anzuwenden.

Die Medikament-Dosimeter-Kombipackung kann aber auch so konstruiert sein, dass der Patient, bzw. Arzt den Anzeigestreifen erst entfernen, z.B. herausziehen oder abbrechen muss, bevor das Arzneimittel zur Gabe entnommen werden kann.

Im Idealfall ist die Medikament-Dosimeter-Kombipackung so konstruiert, dass der Patient, bzw. Arzt, den Test mit dem Anzeigesystem ausführen muss, bevor das Arneimittel in einer bestimmten, aus dem Test resultierenden Dosierung freigegeben wird. Dies lässt sich am besten mit einem Anzeigesystem, das auf Chipbasis funktioniert, bewerkstelligen, da damit quantitative Information direkt auf ein mechanisches System übertragen werden kann, so dass sich z.B. ein mit einem Drehmechanismus versehenes Pillenrad nur um eine definierte Anzahl Umdrehungen öffnen lässt, die dann einer bestimmten Dosierung des in den Tabletten oder Kapseln enthaltenen Arzneistoffs entspricht.

In einer möglichen Ausführungsform ist der erfindungsgemäße Gegenstand als ein mit kleinen Tabletten, Pellets oder Mikropellets gefüllter Container ausgestaltet, der eine definierte Menge/Anzahl Festkörper freigibt. In einer anderen Ausgestaltung der vorliegenden Erfindung enthält der Container eine flüssige Substanz, wobei jeweils ein definiertes Volumen für die orale, sublinguale oder topische Applikation abgegeben wird.

Ein weiteres Beispiel wäre ein Injektor, der auf Grund der vom Chip übertragenen Information nur eine bestimmtes Volumen Arzneistoff z.B. unter die Haut injiziert, oder ein Aerosolgerät, das auf Grund der Information des in das Gerät eingeführten Chips ein bestimmtes Volumen vernebelt, das eine definierte Menge Arzneistoff enthält, die der Patient dann einatmet.

Im Idealfall erfolgt die Übertragung kabellos und in Echtzeit (z.B. Bluetooth Technologie).

### Beispiele

### 1. Antikörper-Therapie

Determinierung einer Responder/Non-responder Situation auf der Basis einer bestimmten Genexpression und entsprechende Entscheidung welcher Therapieansatz optimal ist.

Im Tumorgewebe einer Patientin wird die Expression von HER2 vor Beginn einer Mammakrebstherapie mit Antkörpern gegen HER2 gemessen. Nur wenn HER2 überexprimiert wird, wird die Therapie angesetzt.

### 2. Antioestrogen-Therapie (z.B. mit Tamoxifen) oder andere Tumortherapien

Optimierung der Wirkung unter gleichzeitiger weitestmöglicher Reduzierung der Nebenwirkungen.

In bestimmten Zeitabständen führt die Patientin mit dem bereitgestellten Anzeigesystem ein Oestrogenrezeptorexpressionstest durch. Aus der gemessenen Anzahl Rezeptoren errechnet sich eine individuelle Dosierung. Alternativ wird ein Tumormarker bestimmt (z.B. M2-PK, CEA. MUC-1, etc), der die Unterdrückung oder Ausbreitung der Metastasierung des Tumors anzeigt. Damit wird über den zeitlichen Verlauf einer Langzeitbehandlung immer die optimale Dosierung zur Verfügung gestellt.

### 3. Lipisenkung (Statine)

Kontrolle, bzw. Vermeidung der Nebenwirkungen, bzw. Monitoring des Interaktionspotentials bei Multidrugtherapie (Akut-System).

Die Creatinkinase (CK) oder Elastase aus dem Serum des Patienten wird vor der Einnahme des Lipidsenkers gemessen. Die Dosierung wird erniedrigt oder das Medikament abgesetzt, wenn sich eine erhöhte Rhabdomyolyse (enzymatischer Muskelabbau) aus der gemessenen Konzentration der CK oder Elastase errechnen lässt.

### 4. β - Blocker (Bluthochdruck oder post-MI)

Anpassung der Dosis an eine veränderte Targetexpression, so dass die Arzneimittel-Wirksamkeit in gleicher Stärke erhalten bleibt.

Ein Rezeptorexpressionstest wird durchgeführt, womit eine Veränderung der Anzahl Rezeptoren (up/down-regulation) erkannt und die Dosierung entsprechend angepasst werden kann.

### 5. Antidepressiva (oder andere Cyt. P450 inhibierende oder stimulierende Arzneimittel)

Detektieren von Genexpression oder eines metabolischen/enzymatischen Prozesses, der mit verschiedener Geschwindigkeit ablaufen kann (langsam / schnell) und eine entsprechende Anpassung der Dosierung an die zu erwartende Metabolismusrate erfordert.

Abhängig von der genetischen Prädisposition kann das für den Metabolismus verantwortliche Enzym Cytochrom P450 bei Einnahme von Antidepressiva inhibiert oder besondes aktiv sein. Dies kann zur Inhibition des Metabolismus der eingenommenen Antidepressiva, aber auch anderer Arzneisubstanzen führen. Daraus können gefährlich hohe Plasmaspiegel resultieren. Auf der anderen Seite verhindert ein sehr starker Metabolismus den Aufbau eines wirksamen Blutspiegels. Das Cytochrom P450 eines Patienten wird mit einem Genexpressionschip direkt oder indirect via metabolischer Konversion eines entpsrechenden Substrates charakterisiert. Der Patient wird basierend auf dem Ergebnis als slow / fast metabolizer eingestuft und die Dosis des Antidepressivums entsprechend angepasst.

Aus den besprochenen Beispielen wird deutlich, dass es durchaus denkbar ist, Patienten individuell anstatt nach einem auf statistischen Informationen beruhenden generalisierten, aber viel zu grob gerasterten Behandlungsschema zu behandeln. Die Konsequenz ist eine bessere, dem Einzelfall angepasste Wirksamkeit eines Arzneistoffs, die wegen der Optimierung sowohl der Dosis als auch des Dosierungsintervals im Idealfall auch noch eine signifikante Verringerung der Nebenwirkungen nach sich zieht. Daraus ergibt sich insgesamt sowohl eine höhere Heilungswahrscheinlichkeit und bessere Lebensqualität für den Patienten, als auch eine Verringerung der Gesamtkosten und damit ein positiver ökonomischer Effekt für den Patienten, bzw. das Gesundheitssystem.

In speziellen Fällen ist es darüber hinaus auch möglich; sogenannte Non-responder von vorneherein vor einer ineffektiven (unsinnigen Behandlung), die oft mit gravierenden Nebenwirkungen behaftet ist, zu bewahren.

## Patentansprüche

1. Eine Medikament-Dosimeter-Kombipackung, die in einer Verpackung
a. ein individuell dosierbares Arzneimittel, und
b. ein diagnostisches Anzeigesystem für einen körpereigenen Stoff, Regulationsmechanismus oder Gen oder Indikationssystem, relevant für die Wirkung, Nebenwirkung, Interaktion, Stoffwechsel, Absorption, Verteilung, Metabolismus und Elimination des zu verabreichenden Arzneistoffes
enthält.

2. Die Kombipackung nach Anspruch 1, bei der das diagnostische Anzeigensystem aus einem mit einer Chemikalie imprägnierten Stäbchen, einem mit einer chemischen oder biologischen Indikatorsubstanz beschichteten Vertiefung oder Welle, oder einem mit einer chemischen oder biologischen Indikatorsubstanz beschichteten Chip besteht.

3. Die Kombipackung nach Ansprüchen 1 und 2, wobei die Detektion der gesuchten Information visuell, spektrophotometrisch, fluorometrisch, oder elektronisch sein kann.

4. Die Kombipackung nach Ansprüchen 1 bis 3, bei dem die individuelle Dosierung des Arzneimittels durch mechanische oder elektronische Kalibrierung einer Tablette, eines Tablettenbehälters, einer Flüssigkeit, eines Flüssigkeitsbehälters, eines Spenders, eines Injektors, einer Tube für halb-feste Arzneisubstanzen, oder eines Behälters zur Verneblung von Flüssigkeiten oder Pulvern besteht.

5. Die Kombipackung nach Anspruch 1, mit der die Therapie individuell auf Patienten und deren physiologischen, bzw. pathologischen Zustand abgestimmt wird.

## Claims

1. A medicament/dosing device combined package which contains, in one packaging,
a. a medicament that can be dosed individually and
b. a diagnostic detection system for an endogenous substance, regulation mechanism or gene or indication system that is relevant for the action, side effect, interaction, metabolism, absorption, distribution and elimination of the medicament to be administered.

2. The combined package according to claim 1, in which the diagnostic detection system consists of a strip which is impregnated with a chemical, a depression or well which is coated with a chemical or biological indicator substance, or a chip which is coated with a chemical or biological indicator substance.

3. The combined package according to claims 1 and 2, wherein the detection of the sought information may take place visually, spectrophotometrically, fluorometrically or electronically.

4. The combined package according to claims 1 to 3, in which the individual dosing of the medicament takes place by mechanical or electronic calibration of a tablet, a tablet container, a liquid, a liquid container, a dispenser, an injector, a tube for semi-solid medicaments, or a container for atomising liquids or powders.

5. The combined package according to claim 1, by means of which the therapy is adapted individually to patients and their physiological or pathological condition.

## Revendications

1. Ensemble combiné médicament-dosimètre contenant, dans un emballage
a. un médicament pouvant être dosé individuellement, et
b. un système d'affichage diagnostique destiné à une substance propre au corps, un mécanisme de régulation, un gène ou un système d'indication, important pour l'effet, l'effet secondaire, l'interaction, le métabolisme, l'absorption, la répartition, le métabolisme et l'élimination de la substance médicamenteuse à administrer.

2. Ensemble combiné selon la revendication 1, dans lequel le système d'affichage diagnostique est constitué d'un bâtonnet imprégné d'un produit chimique, d'un creux ou d'une partie en saillie recouvert(e) d'une substance indicatrice chimique ou biologique, ou d'une puce recouverte d'une substance indicatrice chimique ou biologique.

3. Ensemble combiné selon les revendications 1 et 2, dans lequel la détection de l'information recherchée s'effectue de façon visuelle, par spectrophotométrie, par fluorimétrie ou de façon électronique.

4. Ensemble combiné selon les revendications 1 à 3, dans lequel le dosage individuel du médicament s'effectue par calibrage mécanique ou électronique d'un comprimé, d'un récipient de comprimé, d'un liquide, d'un récipient pour liquide, d'un distributeur, d'un injecteur, d'un tube pour substances médicamenteuses semi-solides, ou d'un récipient de nébulisation de liquides ou de poudres.

5. Ensemble combiné selon la revendication 1, au moyen duquel la thérapie peut être adaptée individuellement aux patients et à leur état physiologique et/ou pathologique.
